# EUROPÄISCHE PATENTSCHRIFT

(11) **EP 0 853 757 B1**
(45) Veröffentlichungstag und Bekanntmachung des Hinweises auf die Patenterteilung: **28.02.2001**
(21) Anmeldenummer: 96934509.9
(22) Anmeldetag: 01.10.1996
(51) Int. Cl.: G01K 13/00, A61N 5/00, G01J 1/50

(54) **VORRICHTUNG ZUR ERFASSUNG UND WARNUNG VOR HAUTSCHÄDIGENDER SONNENBESTRAHLUNG**
DEVICE FOR DETECTING AND WARNING AGAINST SKIN-DAMAGING SOLAR RADIATION
DISPOSITIF DE DETECTION ET DE MISE EN GARDE CONTRE LE RAYONNEMENT SOLAIRE PROVOQUANT DES LESIONS CUTANEES

(30) Priorität: 05.10.1995 DE 19537048
(43) Veröffentlichungstag der Anmeldung: 22.07.1998
(73) Patentinhaber: WANFRIED-DRUCK KALDEN GMBH, D-37281 Wanfried (DE); WEBER, Gerhard, Prof.Dr., D-96172 Mühlhausen (DE)
(72) Erfinder: WEBER, Gerhard, D-96172 Mühlhausen (DE)
(74) Vertreter: Hübner, Gerd, Dipl.-Phys.
(86) Internationale Anmeldenummer: EP9604287
(87) Internationale Veröffentlichungsnummer: WO9713129

(56) Entgegenhaltungen:
- EP-A- 0 596 488
- BE-A- 902 327
- CH-A- 532 809
- DE-A- 3 432 387
- DE-A- 4 100 543
- US-A- 4 212 535
- US-A- 4 509 533
- US-A- 4 985 632

## Beschreibung

Die vorliegende Erfindung betrifft eine Vorrichtung zur Erfassung und Warnung vor hautschädigender Sonnenbestrahlung.

Zum besseren Verständnis dieser Erfindung ist deren medizinischer Hintergrund zu erläutern. So wird nach landläufiger Meinung ein Sonnenbrand bei einer zu langen Sonnenbestrahlung der menschlichen Haut durch den UV-Anteil des Sonnenlichtes hervorgerufen. Demzufolge werden zum Schutz vor Sonnenbrand und Hautschädigungen, wie z. B. Hautkrebs, Lichtschutzmittel propagiert, deren sogenannter Lichtschutzfaktor den Faktor angibt, um den die Haut mit gleicher Intensität länger als die ungeschützte Haut bestrahlt werden kann, ohne eine Irritation oder Schädigung zu erfahren. Herkömmliche Lichtschutzmittel weisen daher als UV-Filter wirkende Anteile auf, durch die verhindert werden soll, daß der schädliche UV-Anteil des Sonnenlichtes die menschliche Haut erreicht.

Die Erfahrung hat gezeigt, daß eine vermehrte Anwendung solcher Lichtschutzmittel und stetige Erhöhung deren Lichtschutzfaktors nicht zu einem wirkungsvollen Schutz der Haut führt, was durch eine allseits festgestellte alarmierende Zunahme der Hautkrebse belegbar ist. Es kann daher nicht der UV-A- bzw. -B-Anteil des Sonnenlichtes allein oder in der Hauptsache für solche Hautschädigungen aufgrund Sonnenbestrahlung verantwortlich gemacht werden.

Betrachtet man hinsichtlich dieses Problems die von der Sonne ausgehende Strahlung - die sogenannte "Global-Strahlung" - so ergibt sich für deren prozentuale Zusammensetzung ein UV-Anteil von nur 9 %, wohingegen für die Infrarotstrahlung ein Anteil von 46 % festzustellen ist. Durch die Einbeziehung des wärmespeichernden Anteils des sichtbaren Lichtes erhöht sich dieser Anteil auf 70 %, der als "Wärmeanteil" zu bezeichnen ist.

Wie durch jüngste wissenschaftliche Untersuchungen (siehe den Fachartikel von Prof. Dr. G. Weber "Lichtschutzfaktor erhöht das Risiko Hautkrebs" in hautnah derm 11 (1995), Seiten 139 - 144) belegbar ist, sind Schädigungen der Haut durch Sonnenlicht in erster Linie Folge einer übermäßigen Wärmeeinstrahlung.

Ausgehend von dieser Erkenntnis liegt der Erfindung die Aufgabe zugrunde, eine Vorrichtung zur Erfassung und Warnung vor hautschädigender Sonnenbestrahlung zu schaffen, die auf den wärmetransferierenden Teil des sichtbaren Lichtes und den Infrarot-Anteil der Globalstrahlung anspricht und diesen Wärmeanteil für die der Globalstrahlung ausgesetzte Person erkennbar macht.

Diese Aufgabe wird durch eine Vorrichtung gemäß Anspruch 1 gelöst. Diese Vorrichtung weist demnach einen auf die menschliche Haut auflegbaren Träger, einen in den Träger integrierten Temperatursensor zur Erfassung einer Gleichgewichtstemperatur, die sich aufgrund der Wechselwirkung zwischen der Hauttemperatur der die Vorrichtung benutzenden Person und der Sonnenbestrahlung einstellt, sowie einen Abstandshalter zur Ausbildung eines Abstandes zwischen Temperatursensor und Haut und einen Temperaturindikator zur Sichtbarmachung der Gleichgewichtstemperatur auf.

Die Erfindung geht dabei von der Erkenntnis aus, daß die menschliche Haut - abgesehen von Krankheitsfällen, wie Fieber - physiologisch eine Temperatur im Bereich zwischen 28 ° und 32 °C aufweist. Wird die Haut nun über längere Zeit der Globalstrahlung mit ihrem hohen Wärmeanteil ausgesetzt, so daß die Gefahr einer Hautschädigung besteht, so steigt die Hauttemperatur über den angegebenen Bereich hinaus an, was ein sicheres Indiz dafür ist, daß ein Sonnenbrand und infolge davon schwerwiegendere Hauterkrankungen drohen.

Nun ist die direkte Messung der Hauttemperatur schwierig, wenn dabei gleichzeitig die Globalstrahlung einwirkt.

Bei der erfindungsgemäßen Vorrichtung ist daher der angegebene Temperatursensor zur Erfassung der Gleichgewichtstemperatur vorgesehen, die sich aufgrund der Wechselwirkung zwischen der Hauttemperatur und der Sonnenbestrahlung einstellt. Solange sich die Hauttemperatur in einem unkritischen Bereich befindet, wird also trotz einer hohen Sonnenbestrahlung die sich einstellende Gleichgewichtstemperatur ebenfalls unkritisch sein. Erst wenn sich die Hauttemperatur erhöht, steigt die Gleichgewichtstempeatur durch den Wechselwirkungseinfluß. Durch den ebenfalls vorgesehenen Temperaturindikator zur Sichtbarmachung der Gleichgewichtstemperatur kann deren Anstieg von der die erfindungsgemäßen Vorrichtung tragenden Person erkannt werden. Aufgrund dieses Warnhinweises kann diese Person dann ein Sonnenbad abbrechen, wodurch Hautschädigungen wirkungsvoll vermieden werden.

Um die erfindungsgemäße Vorrichtung besonders preisgünstig zu gestalten und damit als Massenartikel herstellen zu können, ist gemäß Anspruch 2 vorgesehen, den Temperatursensor und den Temperaturindikator gemeinsam in Form eines Folienstückes auszubilden, das mikroverkapselte, temperaturempfindliche Flüssigkristalle aufweist. Derartige Folien mit mikroverkapselten Flüssigkristallen sind üblich und werden als Temperaturindikatoren in einem sehr breiten Anwendungsgebiet eingesetzt. Bekannte Anwendungen sind sogenannte Fieberschnelltest-Streifen, einfache Thermo meter für Kühlschränke, Tiefkühltruhen, Getränkebehälter und -kühler, Thermoskopfolien für thermographische Untersuchung der Blutzirkulation in Extremitäten (z. B. in der Fußsohle und in der Handfläche) usw.

Typische Farben, die die temperaturempfindlichen Flüssigkristalle in Abhängigkeit ihrer Temperatur annehmen, sind z. B. Braun-Rot, Grün und Blau.

Um die Sichtbarmachung der Gleichgewichtstemperatur und insbesondere das Erreichen einer Gleichgewichts-Grenztemperatur bei der erfindungsgemäßen Vorrichtung der sie verwendenden Person augenfällig sichtbar zu machen, können nach Anspruch 3 auf dem Träger Referenz-Farbflächen aufgebracht sein, deren Farbgebung der Farbgebung der Flüssigkristalle bei jeweils einer bestimmten Gleichgewichtstemperatur und insbesondere bei der Gleichgewichts-Grenztemperatur entspricht. Die Person kann also mit einem Blick erkennen, ob die erfaßte und sichtbar gemachte Gleichgewichtstemperatur im unkritischen Bereich, im kritischen Bereich der Gleichgewichts-Grenztemperatur oder im überkritischen Bereich darüber liegt.

Nach Anspruch 4 beträgt die Gleichgewichts-Grenztemperatur vorzugsweise 35 °C bis 36 °C.

Durch die Weiterbildung des Erfindungsgegenstandes gemäß Anspruch 5 kann die erfindungsgemäße Vorrichtung nicht nur die schädliche Wärme, sondern auch die ebenfalls zu Hautschädigungen beitragende UV-A- und -B-Strahlung erfassen. Durch den in Anspruch 6 angegebenen Farb-Indikator, der mittels eines Farbumschlages die Einstrahlung einer bestimmten UV-A- und -B-Leistung indiziert, funktioniert die Vorrichtung auch bei geringeren Außentemperaturen, wie z. B. im Hochgebirge oder auf See, wo die Wärmestrahlung eher eine untergeordnete Rolle spielt.

Die Ansprüche 7 und 8 kennzeichnen vorteilhafte Alternativen für die Ausbildung des den Temperatursensor bzw. -indikator aufnehmenden Trägers, bei dem es sich um ein Armband oder ein auf die Haut applizierbares Pflaster handeln kann. Damit sich dabei eine den tatsächlichen Verhältnissen entsprechende Gleichgewichtstemperatur ausbilden kann, ist in beiden Fällen darauf zu achten, daß sich zwischen dem Träger und der Haut kein Wärme- bzw. Konvektionsstau bildet.

In dieser Hinsicht ist es gemäß Anspruch 9 vorteilhaft, daß der Träger mit Abstandshaltern zur Ausbildung von Spalten zwischen der Haut und dem Träger versehen ist. Nach Anspruch 10 können diese Abstandshalter als Stege ausgebildet sein, deren Höhe etwa 2 mm beträgt.

Anspruch 11 lehrt eine bevorzugte Ausführungsform der Erfindung, bei der die Warnvorrichtung geschützt in einem transparenten, wasserdicht gekapselten Einsatz untergebracht ist, der wiederum in einem mit Hilfe eines Armbandes am Handgelenk einer Person tragbaren Rahmen untergebracht ist. Damit ist die Warnvorrichtung - was das äußere Erscheinungsbild des Gehäuses anbelangt - etwa in der Art einer einfachen LCD-Uhr ausgestaltet.

Weitere Merkmale, Einzelheiten und Vorteile der Erfindung sind der nachfolgenden Beschreibung entnehmbar, in der ein Ausführungsbeispiel des Erfindungsgegenstandes anhand der beigefügten Zeichnungen näher erläutert wird. Es zeigen:
- Fig. 1: eine Draufsicht auf eine Vorrichtung zur Erfassung und Warnung vor hautschädigender Sonnenbestrahlung,
- Fig. 2: eine Ansicht der Unterseite der Vorrichtung gemäß Fig. 1,
- Fig. 3: einen teilweisen schematischen Längsschnitt der Vorrichtung entlang der Schnittlinie III-III nach Fig. 1,
- Fig. 4 und Fig. 5: eine Drauf- und Seitenansicht einer Vorrichtung zur Erfassung und Warnung vor hautschädigender Sonnenbestrahlung in einer alternativen Ausführungsform, und
- Fig. 6: einen Schnitt durch den bei der Vorrichtung gemäß Fig. 4 und 5 verwendeten Einsatz zur wasserdichten Kapselung des Temperatursensors und -indikators.

Wie aus den beigefügten Figuren deutlich wird, ist eine erfindungsgemäße Vorrichtung zur Erfassung und Warnung hautschädigender Sonnenbestrahlung als Armband 1 ausgestaltet, das am Handgelenk einer Person zu tragen ist. Das Armband 1 besteht aus zwei im Umriß deckungsgleichen, im wesentlichen als langgestrecktes Rechteck ausgebildeten Kunststoff-Streifen 2, 3 aus hautverträglichem Material, die beispielsweise durch Laminieren miteinander verbunden sind. Mittig weisen beide Streifen eine elliptische Ausbauchung 4, 5 in ihrem Umriß auf. Ferner sind vor dem einen Ende 6 des Armbandes in Längsrichtung hintereinander zwei durch Stanzen gebildete Zungen 7 vorgesehen, deren freies Ende in Richtung zur mittigen Ausbauchung 4, 5 weist. Am gegenüberliegenden Ende 8 sind zwei quadratische Ausschnitte 9 aus dem Armband 1 ausgestanzt, in die beim Anlegen des Armbandes 1 die Zungen 7 eingeschoben werden können, womit das Armband 1 geschlossen werden kann.

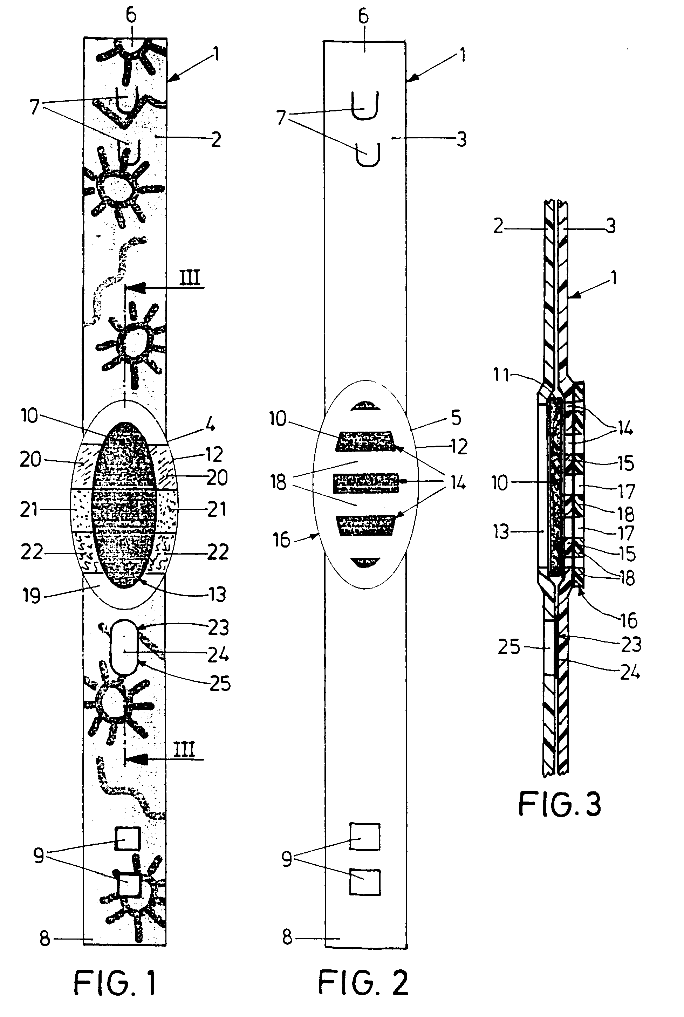

Das Armband 1 dient als auf die menschliche Haut auflegbarer Träger für ein im Bereich der Ausbauchungen 4, 5 der beiden Kunststoff-Streifen 2, 3 eingesetztes Folienstück 10, das mikroverkapselte, temperaturempfindliche Flüssigkristalle aufweist. Das Folienstück 10 ist elliptisch ausgestaltet, wobei sein Rand 11 einige Millimeter innerhalb des Randes 12 der Kunststoff-Streifen 2, 3 im Bereich deren Ausbauchungen 4, 5 verläuft. Der obere Kunststoff-Streifen (Fig. 1) ist oberhalb des Folienstückes 10 ebenfalls mit einem elliptischen Ausschnitt 13 versehen, der etwas kleiner als das Folienstück 10 ist. Durch den Ausschnitt 13 kann das Folienstück 10 mit der Globalstrahlung beaufschlagt werden, ist aber ansonsten zwischen den Kunststoff-Streifen 2, 3 einlaminiert.

Der untere Kunststoff-Streifen 3 (Fig. 2) weist im Bereich des Folienstückes 10 mehrere in Querrichtung verlaufende Ausschnitte 14 auf, deren Enden im Umriß dem Ausschnitt 13 deckungsgleich sind. Zwischen den Ausschnitten 14 im Kunststoff-Streifen 3 bleiben Streben 15 stehen. Auf der der Haut des Trägers zugewandten Unterseite des Kunststoff-Streifens 3 ist im Bereich dessen Ausbauchung 5 ferner ein Abstandshalter 16 vorgesehen, der im Umriß mit der elliptischen Ausbauchung 5 deckungsgleich ist. Mit den Ausschnitten 14 fluchten Aussparungen 17 im Abstandshalter 16, die zwischen sich Stege 18 auf den Streben 15 bilden. Der Abstandshalter 16 mit seinen Stegen 18, die eine Höhe von etwa 2 mm aufweisen, sorgt dafür, daß das Folienstück 10 nicht direkt auf der Haut aufliegt und vermeidet dadurch einen Wärme- und Konvektionsstau.

Im übrigen ist darauf hinzuweisen, daß in Fig. 3 die Dickenverhältnisse bei den einzelnen Bauteilen der Vorrichtung absolut und relativ zueinander nicht maßstabsgetreu sind. Während die Kunststoff-Streifen 2, 3 und das Folienstück 10 eine Dicke von wenigen lOtel Millimeter aufweisen, ist der Abstandshalter 16 etwa 2 mm dick.

Auf dem den Ausschnitt 13 im oberen Kunststoff-Streifen 2 umgebenden, ringelliptischen Randstreifen 19 sind Referenzfarbflächen 20, 21, 22 aufgedruckt, deren Farbgebung der Farbgebung des Folienstückes bei jeweils einer bestimmten Temperatur entspricht. So kann die Referenzfarbfläche 20 braun-rot gefärbt sein, was der Farbgebung der Flüssigkristalle im Folienstück 10 bei einer Temperatur von weniger als 35 °C bis 36 °C entspricht. Die Referenzfarbfläche 21 kann grün - entsprechend einer Temperatur der Flüssigkristalle von 35 °C bis 36 °C - und die Referenzfarbfläche 22 blau - entsprechend einer Temperatur der Flüssigkristalle von mehr als 36 °C - eingefärbt sein. Damit kann die Farbe, die das Folienstück 10 mit seinen Flüssigkristallen bei einer bestimmten Gleichgewichtstemperatur im Einsatz annimmt, vom Benutzer der Vorrichtung einer bestimmten Temperatur zugeordnet und es können daraus die entsprechenden Schlußfolgerungen gezogen werden. Dies wird im einzelnen noch näher beschrieben. Zu ergänzen ist, daß das Folienstück beim Temperaturen von mehreren Grad unter oder über 35 °C bis 36 °C - also beispielsweise unter 32 °C und über 39 °C - eine schwarze Färbung annimmt.

Die erfindungsgemäße Vorrichtung weist ferner einen Farbindikator 23 ebenfalls in Form eines zwischen den beiden Kunststoff-Streifen 2, 3 einlaminierten Folienplättchens 24 auf, das von oben über eine langlochförmige Aussparung 25 im oberen Kunststoff-Streifen 2 mit der Globalstrahlung beaufschlagt werden kann. Der Farbindikator 23 erfaßt die von der Globalstrahlung eingestrahlte Menge an UV-A- und -B-Licht und indiziert mittels eines Farbumschlages die Einstrahlung einer bestimmten UV-A-und -B-Lichtmenge. Damit kann die erfindungsgemäße Vorrichtung nicht nur den Wärmeanteil der eingestrahlten Globalstrahlung, sondern auch den hinsichtlich Hautschädigungen ebenfalls relevanten Anteil an UV-A- und -B-Licht erfassen und sichtbar machen.

Im übrigen ist zu ergänzen, daß das Armband und insbesondere das Folienstück 10 mit den mikroverkapselten Flüssigkristallen und der Farbindikator 23 wasserunempfindlich sind, so daß die erfindungsgemäße Vorrichtung auch im Wasser verwendbar ist. Hier spielt die Wärmestrahlung keine Rolle, jedoch kann eine Schädigung durch den UV-Anteil der Global-strahlung auftreten. Dieser wird - wie erörtert - über den Farbindikator 23 erfaßt, so daß auch unter Wasser eine entsprechende Warnung vor einem Sonnenbrand von der erfindungsgemäßen Vorrichtung ausgegeben werden kann.

Die Funktionsweise der erfindungsgemäßen Vorrichtung zur Erfassung und Warnung vor hautschädigender Sonnenbestrahlung wird im folgenden anhand eines beispielhaften typischen Verlauf eines Sonnenbades näher erläutert:
- Es wird davon ausgegangen, daß die die Vorrichtung benutzende Person das Armband 1 anlegt und sich in die Sonne begibt. Die Hauttemperatur beträgt 28 °C, die von der Globalstrahlung hervorgerufene Temperatur 30 °C. Im Folienstück 10 stellt sich eine Gleichgewichtstemperatur von z. B. 29 °C ein, so daß die Flüssigkristalle keinen Farbumschlag zeigen
   und das Folienstück 10 schwarz eingefärbt bleibt. Gleichzeitig soll der Farbindikator 23 keinen Farbumschlag zeigen.
   Dadurch wird signalisiert, daß ein kritischer Meßwert für den Benutzer noch nicht erreicht ist.
- Anschließend soll sich die durch die Globalstrahlung hervorgerufene Temperatur auf 40 °C erhöhen. Solange noch keine Hautirritationen aufgrund des Wärmeanteils der Globalstrahlung auftreten, bleibt die Hauttemperatur bei 28 °C und es stellt sich eine höhere Gleichgewichtstemperatur im Folienstück 10 z. B. von 34 °C ein. Die Flüssigkristalle im Folienstück 10 zeigen einen Farbumschlag nach braun-rot, was der Benutzer durch einen Vergleich mit den Referenzfarbflächen 20, 21, 22 noch als unkritischen Zustand erkennen kann, sofern der UV-Farbindikator 23 nach wie vor keinen Umschlag zeigt.
- Im folgenden soll die vom Wärmeanteil der Globalstrahlung hervorgerufene Temperatur bei 40 °C bleiben und die Hauttemperatur aufgrund der Bestrahlung auf 32 °C ansteigen. Die sich im Folienstück 10 einstellende Gleichgewichtstemperatur liegt dann z. B. bei 36 °C, so daß die Flüssigkristalle im Folienstück 10 eine grüne Farbgebung annehmen. Der Benutzer kann diese kritische Situation wiederum durch einen Vergleich der Farbgebung des Folienstückes 10 mit den Referenzfarbflächen 20, 21, 22 wahrnehmen und sich zu seinem Schutz in den Schatten begeben.
- Tut er letzteres nicht, so steigt die Hauttemperatur beispielsweise auf sehr kritische 36 °C an, so daß sich die Gleichgewichtstemperatur auf z. B. 38 °C einstellt. Das Folienstück 10 nimmt eine blaue Farbgebung an, womit seine Temperatur wiederum durch Vergleich mit den Referenz-farbflächen 20, 21, 22 vom Benutzer der Vorrichtung erfaßbar und dieses als Warnhinweis vor der herrschenden äußerst kritischen Situation verstanden wird. Er muß sich nun unbedingt in den Schatten begeben, um einen Sonnenbrand aufgrund des Wärmeanteils der Globalstrahlung zu vermeiden.
- Für den Fall, daß die erfindungsgemäße Vorrichtung auch im Hochgebirge verwendet wird, wo der Wärmeanteil der Globalstrahlung in der Regel unkritisch und eine Gefährdung in erster Linie durch den UV-A-und -B-Anteil der Globalstrahlung hervorgerufen wird, spielt der Farbindikator 23 hinsichtlich einer Warnung vor Hautschädigungen die entscheidende Rolle. Dieser schlägt bei einem entsprechend hohen UV-Anteil in seiner Farbgebung um und signalisiert dem Benutzer so, daß der kritische Punkt erreicht ist und er eine Hautschädigung befürchten muß.

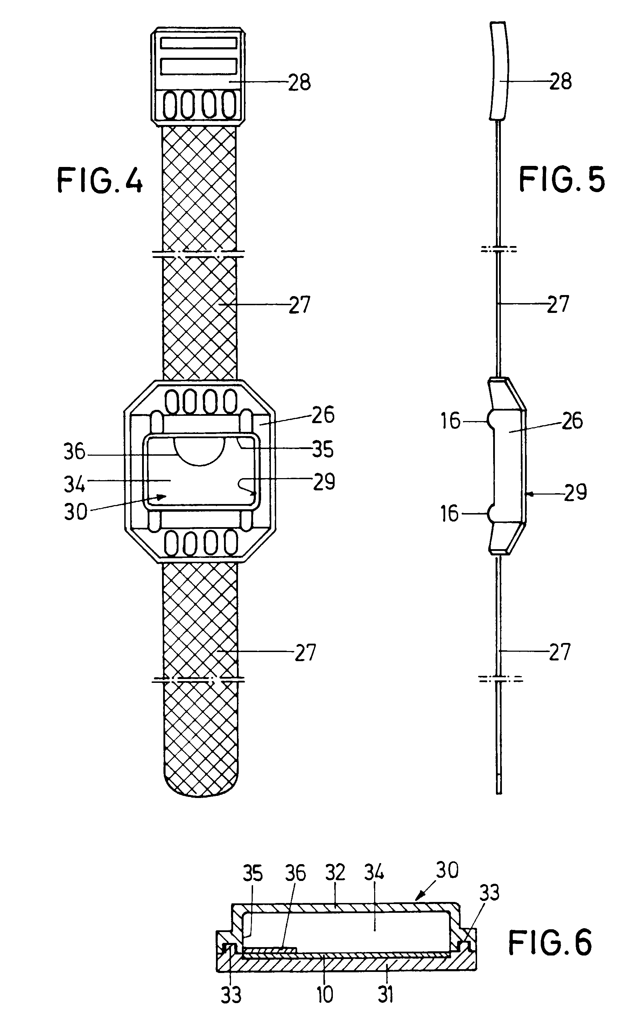

In den Fig. 4 bis 6 ist eine weitere Ausführungsform des Erfindungsgegenstandes dargestellt. Hierbei ist als Träger ein als Kunststoff-Spritzgußteil ausgestalteter Rahmen 26 vorgesehen, der mittels eines üblichen textilen Armbandes 27 mit Verschluß 28 von einer Person am Handgelenk zu tragen ist. An der Unterseite des Rahmens 26 sind wiederum Abstandshalter 16 vorgesehen, um einen Wärmestau darunter zu vermeiden. In der Öffnung 29 des Rahmens 26 ist ein flacher, quaderförmiger Einsatz 30 befestigt, der aus transparentem, UV- und IR-durchlässigen Material hergestellt ist. Wie aus Fig. 6 erkennbar ist, weist der Einsatz 30 einen Boden 31 und einen darauf sitzenden Deckel 32 auf, die im Bereich einander zugewandter Randflansche 33 wasserdicht ultraschallverschweißt sind. Im Innenraum 34 des Einsatzes 30, und zwar auf dem Boden 31 ist in diesem Falle das Folienstück 10 mit den mikroverkapselten, temperaturempfindlichen Flüssigkristallen aufgebracht, das in der erörterten Weise als Temperatursensor und - indikator wirkt. Entlang einer Längsseite 35 ist mittig ferner ein halbkreisförmiger UV-Sensor 36 auf das Folienstück 10 aufgesetzt.

## Patentansprüche

1. Vorrichtung zur Erfassung und Warnung vor hautschädigender Sonnenbestrahlung mit
- einem auf die menschliche Haut auflegbaren Träger (1),
- einem in den Träger (1) integrierten Temperatursensor (10) zur gleichzeitigen Erfassung der von der Haut abgestrahlten Temperatur und der aufgrund der Global-Strahlung hervorgerufenen Temperatur unter Einstellung des Temperatursensors (10) auf eine Gleichgewichtstemperatur, die sich aufgrund der Wechselwirkung zwischen der Hauttemperatur und dem Wärmeanteil der Sonnenbestrahlung einstellt,
- einem Abstandshalter (16) zur Ausbildung eines Abstandes zwischen Temperatursensor (10) und Haut, und
- einem Temperaturindikator (10) zur Sichtbarmachung der Gleichgewichtstemperatur.

2. Vorrichtung nach Anspruch 1, **dadurch gekennzeichnet, daß** der Temperatursensor und der Temperaturindikator gemeinsam in Form eines Folienstückes (10) enthaltend mikroverkapselte, temperaturempfindliche Flüssigkristalle ausgebildet sind, deren variable Farbgebung abhängig von der Gleichgewichtstemperatur ist.

3. Vorrichtung nach Anspruch 2, **dadurch gekennzeichnet, daß** auf dem Träger (1) Referenz-Farbflächen (20, 21, 22) aufgebracht sind, deren Farbgebung der Farbgebung der Flüssigkristalle bei jeweils einer bestimmten Gleichgewichtstemperatur, insbesondere einer Gleichgewichts-Grenztemperatur entspricht, ab der eine Hautschädigung durch den Wärmeanteil der Sonnenbestrahlung zu erwarten ist.

4. Vorrichtung nach Anspruch 3, **dadurch gekennzeichnet, daß** die Gleichgewichts-Grenztemperatur 35 °C bis 36 °C beträgt.

5. Vorrichtung nach einem der Ansprüche 1 bis 4, **dadurch gekennzeichnet, daß** in den Träger (1) ein UV-Sensor (23) für die von der Sonenbestrahlung eingestrahlte Leistung an UV-A- und -B-Licht integriert ist.

6. Vorrichtung nach Anspruch 5, **dadurch gekennzeichnet, daß** der UV-Sensor ein Farbindikator (23) ist, der mittels eines Farbumschlages die Einstrahlung einer bestimmten UV-A- und -B-Lichtmenge indiziert.

7. Vorrichtung nach einem der Ansprüche 1 bis 6, **dadurch gekennzeichnet, daß** der Träger als um das Handgelenk anlegbares Armband (1) ausgebildet ist.

8. Vorrichtung nach einem der Ansprüche 1 bis 6, **dadurch gekennzeichnet, daß** der Träger als auf die Haut applizierbares, hinterlüftbares Pflaster ausgebildet ist.

9. Vorrichtung nach einem der Ansprüche 1 bis 8, **dadurch gekennzeichnet, daß** der Träger (1) auf seiner der Haut zugewandten Seite zumindest im Bereich des Temperatursensors (10) mit Abstandshaltern (16) zur Ausbildung von Spalten zwischen Haut und Träger (1) versehen ist.

10. Vorrichtung nach Anspruch 9, **dadurch gekennzeichnet, daß** die Abstandshalter (16) als Stege (18) ausgebildet sind, deren Höhe etwa 2 mm beträgt.

11. Vorrichtung nach einem der Ansprüche 1 bis 6, 9 oder 10, **dadurch gekennzeichnet, daß** der Träger als Rahmen (26) ausgebildet ist, der mit Hilfe eines Armbandes (27) am Handgelenk einer Person tragbar ist, wobei der Temperatursensor und Temperaturindikator in Form eines Flüssigkristall-Folienstückes (10) in einem transparenten, wasserdicht gekapselten Einsatz (30) im Rahmen (26) untergebracht sind.

12. Vorrichtung nach Anspruch 11, **dadurch gekennzeichnet, daß** ein UV-Sensor (36) auf dem Folienstück (10) im Einsatz (30) angeordnet ist.

## Claims

1. A device for the detection and warning of skin-irritant exposure to sunlight, comprising
- a carrier (1) to be placed on the human skin,
- a temperature sensor (10) integrated in the carrier (1) for the simultaneous detection of the temperature radiated by the skin and of the temperature caused by the global irradience, the temperature sensor (10) adjusting itself to an equilibrium temperature which will establish owing to the interaction of the skin temperature and the heat portion of solar radiation,
- a spacer (16) for a distance to form between the temperature sensor (10) and the skin, and
- a temperature indicator (10) for rendering the equilibrium temperature perceptible.

2. A device according to claim 1, **characterized in that** the temperature sensor and the temperature indicator jointly are in the form of a piece of film (10) containing microencapsuled, temperature-sensitive liquid crystals, the variably coloring of which depends on the equilibrium temperature.

3. A device according to claim 2, **characterized in that** reference color areas (20, 21, 22) are applied to the carrier (1), the coloring of each of which corresponds to the coloring of the liquid crystals at a certain equilibrium temperature, in particular to an equilibrium temperature limit from which on damages to the skin by the heat percentage of solar radiation are to be expected.

4. A device according to claim 3, **characterized in that** the equilibrium temperature limit is 35°C to 36°C.

5. A device according to one of claims 1 to 4, **characterized in that** a UV sensor (23) for the power of UV-A and UV-B light irradiated by solar radiation is integrated in the carrier (1).

6. A device according to claim 5, **characterized in that** the UV sensor is a color indicator (23), indicating the irradiation of a certain amount of UV-A and UV-B light by means of a change in color.

7. A device according to one of claims 1 to 6, **characterized in that** the carrier is a wristlet (1) to be put around the wrist.

8. A device according to one of claims 1 to 6, **characterized in that** the carrier is a plaster, which can be applied to the skin and to the back of which air can be admitted.

9. A device according to one of claims 1 to 8, **characterized in that** at least in the vicinity of the temperature sensor (10), the side of the carrier (1) that is turned towards the skin is provided with spacers (16) for gaps to form between the skin and the carrier (1).

10. A device according to claim 9, c**haracterized in that** the spacers (16) are ribs (18), the height of which is approximately 2 mm.

11. A device according to one of claims 1 to 6, 9 or 10, **characterized in that** the carrier is a frame (26) which is wearable on a person's wrist with the aid of a wrist band (27), the temperature sensor and temperature indicator in the form of a piece of film (10) being lodged in a transparent insert (30) encapsuled to be waterproof in the frame (26).

12. A device according to claim 11, **characterized in that** a UV sensor (36) is disposed on the piece of film (10) in the insert (30).

## Revendications

1. Dispositif pour la détection et l'avertissement contre le rayonnement solaire provoquant des lésions cutanées, lequel dispositif comporte :
- un support (1) pouvant être posé sur la peau humaine,
- un capteur de température (10) qui est intégré dans le support (1) et qui détecte simultanément la température dégagée par la peau et la température due à la radiation globale, le capteur de température (10) étant réglé sur une température d'équilibre qui s'installe en raison de l'interaction entre la température de la peau et la part de chaleur du rayonnement solaire,
- un élément d'écartement (16) pour former une distance entre le capteur de température (10) et la peau, et
- un indicateur de température (10) pour visualiser la température d'équilibre.

2. Dispositif selon la revendication 1, caractérisé en ce que le capteur de température et l'indicateur de température sont conçus en commun sous la forme d'un élément en feuille (10) contenant des cristaux liquides microencapsulés et sensibles à la température dont la coloration variable dépend de la température d'équilibre.

3. Dispositif selon la revendication 2, caractérisé en ce que sont disposées sur le support (1) des surfaces colorées de référence (20, 21, 22) dont la coloration correspond à la coloration des cristaux liquides à chaque fois à une température d'équilibre déterminée, notamment à une température d'équilibre limite, à partir de laquelle il faut s'attendre à une lésion cutanée due à la part de chaleur du rayonnement solaire.

4. Dispositif selon la revendication 3, caractérisé en ce que la température d'équilibre limite vaut de 35 °C à 36 °C.

5. Dispositif selon l'une des revendications 1 à 4, caractérisé en ce qu'un capteur UV (23) est intégré dans le support (1) pour la puissance en lumière UV-A et UV-B incidente due au rayonnement solaire.

6. Dispositif selon la revendication 5, caractérisé en ce que le capteur UV est un indicateur de couleur (23) qui indique au moyen d'un changement de couleur le rayonnement incident d'une quantité déterminée de lumière UV-A et UV-B.

7. Dispositif selon l'une des revendications 1 à 6, caractérisé en ce que le support est conçu comme un bracelet (1) pouvant être mis autour du poignet.

8. Dispositif selon l'une des revendications 1 à 6, caractérisé en ce que le support est conçu comme une pièce pouvant être appliquée sur la peau et aérée par l'arrière.

9. Dispositif selon l'une des revendications 1 à 8, caractérisé en ce que le support (1) est muni, sur son côté proche de la peau et au moins dans la zone du capteur de température (10), d'éléments d'écartement (16) pour former des espaces entre la peau et le support (1).

10. Dispositif selon la revendication 9, caractérisé en ce que les éléments d'écartement (16) sont conçus comme des traverses (18) dont la hauteur vaut 2 mm environ.

11. Dispositif selon l'une des revendications 1 à 6 et 9 ou 10, caractérisé en ce que le support est conçu comme un cadre (26) qui peut être porté à l'aide d'un bracelet (27) au poignet d'une personne, le capteur de température et l'indicateur de température étant agencés dans le cadre (26) sous la forme d'un élément en feuille à cristaux liquides (10) dans un élément inséré (30) transparent et capsulé de façon à être étanche à l'eau.

12. Dispositif selon la revendication 11, caractérisé en ce qu'un capteur UV (36) est agencé sur l'élément en feuille (10) dans l'élément inséré (30).
